(19) Europäisches Patentamt
European Patent Office
Office européen des brevets

(11) Veröffentlichungsnummer: **0 030 609**
**B1**

(12) **EUROPÄISCHE PATENTSCHRIFT**

(45) Veröffentlichungstag der Patentschrift: **23.02.83**

(51) Int. Cl.³: **C 07 C 103/38,** C 07 C 103/365, C 07 D 207/27, C 07 D 205/08, C 07 C 87/10, C 07 C 102/00

(21) Anmeldenummer: **80106566.5**

(22) Anmeldetag: **25.10.80**

(54) **Verfahren zur Herstellung von N-alpha-Alkoxyalkyl-carbonsäureamiden sowie einige Vertreter dieser Verbindungsklasse und deren Folgeprodukte.**

(30) Priorität: **03.11.79 DE 2944456**

(43) Veröffentlichungstag der Anmeldung: **24.06.81 Patentblatt 81/25**

(45) Bekanntmachung des Hinweises auf die Patenterteilung: **23.02.83 Patentblatt 83/8**

(84) Benannte Vertragsstaaten: **BE CH DE FR GB IT LI NL**

(56) Entgegenhaltungen:
**DE-A-2 063 017**
**DE-A-2 113 338**

**„Synthesis", Bd. 1971, Nr. 5, Stuttgart, G. L. Isele *et al.* „N-Alkylierung von Carbonsäureamiden", S. 266 bis 267**

(73) Patentinhaber: **HOECHST AKTIENGESELLSCHAFT, Postfach 80 03 20, D-6230 Frankfurt/Main 80 (DE)**

(72) Erfinder: **Schmidt, Erwin, Dr., Am Flachsland 26, D-6233 Kelkheim (Taunus) (DE)**

**EP 0 030 609 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von N-α-Alkoxyalkylcarbonsäureamiden sowie einige Vertreter dieser Verbindungsklasse und deren Folgeprodukte

N-α-Alkoxyalkylcarbonsäureamide sind wertvolle Zwischenprodukte auf verschiedenen Sachgebieten, insbesondere auf dem Polymerengebiet. Zur Herstellung von Polymeren wird aus den N-α-Alkoxyalkylcarbonsäureamiden zunächst nach bekannten Methoden Alkohol zu den entsprechenden N-Alkenyl- bzw. N-Vinylcarbonsäureamiden abgespalten, die dann ihrerseits zu Homo- und Mischpolymerisaten mit interessanten und vielseitigen anwendungstechnischen Eigenschaften polymerisiert werden können („Ullmanns Enzyklopädie der technischen Chemie", 3. Aufl., Bd. 14, S. 261-264).

Zur Herstellung von N-Alkoxyalkylcarbonsäureamiden ist eine Reihe von Methoden bekannt. Sekundäre N-α-Alkoxyalkylcarbonsäureamide — d.s. also solche Carbonsäureamide, welche am Stickstoff noch ein freies Wasserstoffatom tragen — werden hauptsächlich nach elektrochemischen Verfahren hergestellt. Ein solches ist z.B. beschrieben in der DE-OS Nr. 2113338. Danach werden u.a. N-Alkylcarbonsäureamide mit Alkoholen unter Verwendung bestimmter Leitsalze zu den entsprechenden N-α-Alkoxyalkylcarbonsäureamiden alkoxyliert. Nach diesem Verfahren sollen zwar auch N-Dialkylcarbonsäureamide zu den entsprechenden N-α-Alkoxyalkyl-N-alkylcarbonsäureamiden — also tertiären N-α-Alkoxyalkylcarbonsäureamiden (ohne freien Wasserstoff am Amidstickstoff) — alkoxyliert werden können, doch eignet sich das Verfahren in erster Linie nur für die Herstellung sekundärer N-α-Alkoxyalkylcarbonsäureamide, weil im Falle von zwei Alkylgruppen am Amidstickstoff die Alkoxylierung kaum selektiv nur an einer Alkylgruppe ansetzt und das entstehende Gemisch dann schwer auftrennbar ist.

Die elektrochemische Herstellung von sekundären N-α-Alkoxyäthylcarbonsäureamiden aus N-α-Carboxyäthylcarbonsäureamiden ist beschrieben in der DE-OS Nr. 2336976; das Verfahren erfordert jedoch erst die Bereitstellung der als Ausgangsprodukte notwendigen N-α-Carboxyäthylcarbonsäureamide, was einen gewissen Aufwand bedeutet.

Besser speziell zur Herstellung von sekundären N-Alkoxyäthylcarbonsäureamiden eignet sich das elektrochemische Verfahren der anodischen Alkoxylierung von sekundären N-Äthylcarbonsäureamiden gemäss BE-PS Nr. 837906; das Verfahren arbeitet mit einer gewissen Mindeststrommenge und ganz bestimmten Leitsalzen.

Weiterhin wurde vorgeschlagen (EP-A Nr. 0019226), sekundäre N-Alkoxyäthylcarbonsäureamiden mit einem Alkohol in einer Elektrolysezelle mit glasartigem Kohlenstoff als Anodenmaterial und mindestens einem Alkali- und/oder Tetraalkylammoniumalkosulfat als Leitsalz herzustellen.

Die geschilderten elektrochemischen Verfahren arbeiten zwar relativ sauber und mit guten Ausbeuten, doch ist der mit ihrer Durchführung verbundene technische Aufwand nicht unerheblich. Ausserdem gestaltet sich oft die Rückgewinnung der notwendigen, oft recht beträchtlichen Leitsalzmengen — was aus Gründen den Umweltschutzes erforderlich ist — nicht ganz leicht.

Tertiäre N-α-Alkoxyalkylcarbonsäureamide werden aus den bei der Besprechung der DE-OS Nr. 2113338 genannten Gründen kaum auf rein elektrochemischem Weg, sondern hauptsächlich nur chemisch oder teils elektrochemisch und teils chemisch, hergestellt. Eine rein chemische Methode besteht in der Umsetzung von sekundären N-Alkylcarbonsäureamiden mit Acetalen oder Halbacetalen [„Chemische Berichte" *99*, 2127 (1966); DE-PS Nr. 1273533]. Die Ausbeuten sind hier insbesondere bei den geradkettigen Produkten jedoch nicht immer befriedigend. So wird etwa in Beispiel 11 der DE-PS Nr. 1273533 (Umsetzung von N-Methylacetamid mit Acetaldehyddiäthylacetal zu N-α-Äthoxyäthyl-N-methylacetamid) nur eine Ausbeute von 26% angegeben.

Nach einem erst vor kurzem vorgeschlagenen gemischt elektrochemisch-chemischen Verfahren (EP-A Nr. 0019225) werden N-Äthylcarbonsäureamide mit Alkoholen zuerst zu den entsprechenden N-α-Alkoxyäthylcarbonsäureamiden anodisch alkoxyliert, und diese dann einer Alkylierung z.B. mittels eines Alkylhalogenids in alkalischem Medium nach den im Prinzip für derartige Alkylierungen bekannten Methoden („Synthesis" 1971, 966; „Synthesis" 1976, 113 ff.; „Z. Chem." 17, 1977, 260) unterworfen. Obwohl dieses Verfahren recht vorteilhaft und günstig ist, erscheint es doch insbesondere wegen des zur Durchführung der elektrochemischen Verfahrensstufe erforderlichen Aufwands noch weiter verbesserungsbedürftig.

Es ist in den letzten Jahren auch bekannt geworden, eine Lactam — nämlich das Pyridon-2 — am Stickstoff mit einem cyclischen α-Halogenalkyläther — nämlich α-Chlortetrahydrofuran — am Stickstoff zu alkylieren („Tetrahedron Letters" 1976, 1725 bis 1728, siehe insbesondere Beispiel 17 in der Tabelle auf Seite 1727). Die Reaktion lässt sich durch folgende Gleichung wiedergeben:

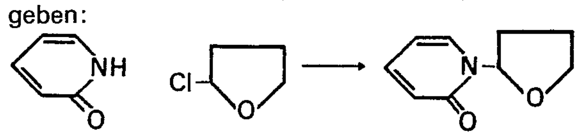

Pyridon-2 α-Chlortetrahydrofuran

Pyridon-2 ist jedoch wegen der Möglichkeit der Ausbildung eines aromatischen Systems kein normales Lactam. Die iso- und mesomeren Formen des Pyridon-2 sind laut L.F. Fieser und M. Fieser „Lehrbuch der organischen Chemie", Verlag Chemie, 1965, S. 1446:

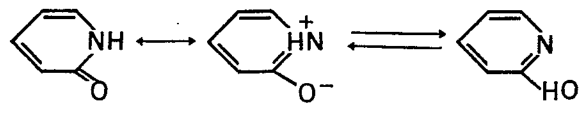

Wegen des besonderen, schon quasi-aromatischen Charakters des Pyridon-2 lässt sich auch die Reaktion mit α-Halogentetrahydrofuran nicht auf normale Carbonsäureamide übertragen, wie durch eigene Versuche bestätigt wurde. Bei diesen Versuchen gelang z.B. die Alkylierung von N-Methylacetamid mit α-Halogentetrahydrofuran nicht.

Ähnliche Reaktionen wie diejenige zwischen Pyridon-2 und α-Chlortetrahydrofuran sind auch aus der Nukleotidchemie bekannt, wo ebenfalls Lactam-ähnliche Verbindungen, die wie das Pyridon-2 zur Ausbildung eines aromatischen Systems befähigt sind, wie z.B. Cytosin, Guanin, Thymin etc. mit α-Halogenzuckern umgesetzt werden, z.B.

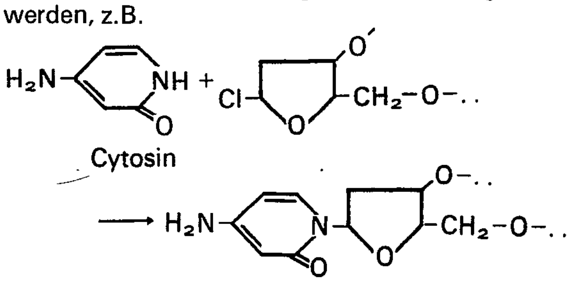

Cytosin

Für die Herstellung etwa von N-α-Alkoxyalkylderivaten normaler Carbonsäureamide (einschliesslich der cyclischen Carbonsäureamide = Lactame) lässt sich diesen Reaktionen nichts entnehmen.

In dem Bestreben, die bekannten Verfahren zur Herstellung von N-α-Alkoxyalkylcarbonsäureamiden weiter zu verbessern, wurde nun gefunden, dass sich dieses Ziel — ausgehend von primären oder sekundären Carbonsäureamiden — dadurch erreichen lässt, dass man primäre oder sekundäre Amide aliphatischer, araliphatischer oder aromatischer Carbonsäuren oder cyclische Carbonsäureamide (Lactame), welche nicht zur Ausbildung eines aromatischen Systems befähigt sind, mit offenkettigen α-Halogenäthern einer C-Atomzahl von mindestens 3 pro Molekül in Gegenwart von tertiären Aminen umsetzt.

Das Gelingen dieser Reaktion mit den dabei erzielten durchweg guten bis sehr guten Ausbeuten war ausserordentlich überraschend, da man — weil die aus „Tetrahedron Letters" 1976, 1725 ff. bekannte Umsetzung von Pyridon-2 mit α-Chlortetrahydrofuran nicht mehr gelingt, wenn man das Pyridon-2 durch ein normales Carbonsäureamid wie z.B. N-Methalacetamid ersetzt — nicht erwarten konnte, dass Versuchen einer N-Alkylierung von normalen Carbonsäureamiden mit α-Halogenalkyläthern überhaupt ein Erfolg beschieden sein konnte. Dass dieser Erfolg tatsächlich eintritt, wenn man anstelle des cyclischen α-Chlortetrahydrofurans offenkettige α-Halogenalkyläther verwendet, war in keiner Weise auch nur annähernd vorhersehbar.

Die bekannten N-Alkylierungen von Carbonsäureamiden mit normalen Halogenalkylen, wie z.B. Chloräthan etc., können mit dieser Reaktion nicht verglichen werden, da normale Halogenalkyle und α-Halogenalkyläther verschiedene Stoffklassen mit verschiedenen Eigenschaften und Reaktivitäten sind.

Als Ausgangsverbindungen für das Verfahren dienen im Prinzip alle möglichen primären oder sekundären Amide aliphatischer, araliphatischer oder aromatischer Carbonsäuren oder cyclische Carbonsäureamide (Lactame), welche nicht — wie etwa Pyridon-2 oder Cytosin etc. — zur Ausbildung eines aromatischen Systems befähigt sind. Konkrete Beispiele solcher Ausgangsverbindungen sind:

$HCONH_2$
$HCONHCH_3$
$HCONHC_3H_7(n)$
$CH_3CONHCH_3$
$(CH_3O)_2CH-CONH_2$
$CH_3CONHC_4H_9(i)$
$ClCH_2CONHC_2H_5$
$Cl_2CHCONH_2$
$Cl_3CCONHCH_3$
$BrCH_2CONHCH_3$
$C_2H_5CONHCH_3$
$C_3H_7CONHC_2H_5$
$C_7H_{15}CONHCH_3$
$CH_3OCH_2CONHCH_3$
$C_6H_5OCH_2CONHCH_3$
$CH_3OOC-CH_2-CONH_2$
$C_2H_5OOC-(CH_2)_4-CONH_2$
$CH_3OOC-CH=CH-CONH_2$

$C_6H_5-CH_2CONHCH_3$
$p-ClC_6H_4-CH_2CONHCH_3$

$C_6H_5CONHC_2H_5$
$n-CH_3C_6H_4CONHCH_3$

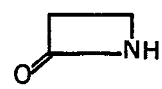
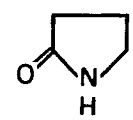

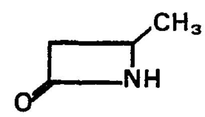
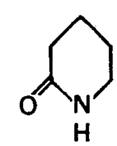

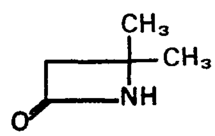
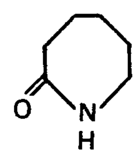

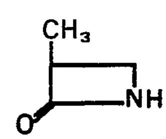
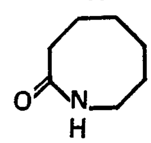
etc.

Bevorzugte Ausgangsverbindungen sind die unter die nachstehende Formel I fallenden primären und sekundären Carbonsäureamide (einschliesslich Lactame):

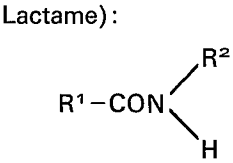
(I)

worin

$R^1$ = H oder ggf. durch reaktionsinerte Gruppen substituiertes $C_1$ bis $C_4$-Alkyl,

$R^2$ = H oder $C_1$ bis $C_3$-Alkyl, oder

$R^1$ + $R^2$ = zusammen eine — ggf. durch reaktionsinerte Gruppen substituierte — Alkylengruppe mit 2 bis 6 C-Atomen in der Kette.

Mit reaktionsinerten Gruppen sind solche Gruppen gemeint, welche unter den angewandten Reaktionsbedingungen keine Reaktion eingehen, also z.B. Alkylgruppen (vorzugsweise $C_1$ bis $C_4$-Alkyl), Alkoxygruppen (vorzugsweise ebenfalls mit 1 bis 4 C-Atomen), die Phenoxygruppe, F, Cl, Br etc.

Besonders bevorzugte primäre und sekundäre Carbonsäureamide sind solche Verbindungen der Formel I mit

$R^1$ = H, $CH_3$, $C_2H_5$ und $R^2$ = H, $CH_3$, $C_2H_5$

Als offenkettige α-Halogenalkyläther einer C-Atomzahl von mindestens 3 pro Molekül eignen sich in beispielhafter Weise:

$CH_3-CHCl-O-CH_3$

$CH_3-CHCl-O-C_2H_5$

$CH_3-CHCl-O-C_3H_7(n)$

$CH_3-CHCl-O-C_4H_9(i)$

$C_2H_5-CHCl-O-CH_3$

$(i\text{-})C_3H_7-CHCl-O-CH_3$

$CH_3-CHCl-O-CH_2-C_6H_5$

$CH_3-CHBr-O-C_2H_5$ etc.

Bevorzugte offenkettige α-Halogenalkyläther sind die unter die nachstehende Formel II fallenden Verbindungen:

$$X-\underset{\displaystyle OR^4}{\underset{|}{CH}}-R^3 \qquad (II)$$

worin

$R^3$ = $C_1$ bis $C_4$-Alkyl,

$R^4$ = ebenfalls $C_1$ bis $C_4$-Alkyl, und

X = Cl oder Br, vorzugsweise Cl.

Diese α-Halogenalkyläther sind nach bekannten Methoden zugänglich, z.B. durch:

Anlagerung von Halogenwasserstoff an Vinyläther,

α-Chlorierung von Dialkyläthern,

Umsetzung von Acetalen mit Säurechloriden, oder

Umsetzung von Aldehyden mit Alkoholen und Halogenwasserstoff.

Hierbei ist die letztgenannte Methode wegen der einfachen Ausgangsmaterialien und Durchführung sowie der guten Ausbeuten bevorzugt. Die dieser Methode zugrundeliegende Reaktionsgleichung ist (für die Herstellung der bevorzugten α-Halogenalkyläther der Formel II):

$$R^3CHO + R^4OH + HX \rightarrow X-\underset{\displaystyle OR^4}{\underset{|}{CH}}-R^3 + H_2O$$

In den Formeln besitzen die Reste $R^3$, $R^4$ und X die gleiche Bedeutung wie in Formel II.

Als tertiäre Amine sind für das erfindungsgemässe Verfahren im Prinzip alle möglichen tertiären Amine einsetzbar wie z.B.:

$(CH_3)_3N$

$(C_2H_5)_3N$

$(CH_3)_2N-CH_2-CH_2N(CH_3)_2$

$(CH_3)_2N-(CH_2)_2-N(CH_3)-(CH_2)_2-N(CH_3)_2$

permethylierte Polyäthylenimine,

$(CH_3)_2N-CH_2-COOC_4H_9$

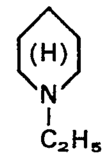
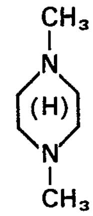

Diazabicyclooctan (DABCO),

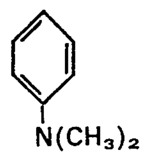
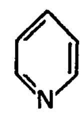
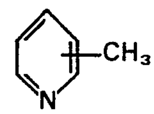
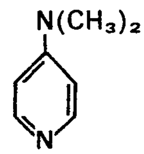

basische Ionenaustauscher mit tert.-Aminogruppen etc.

Bevorzugte tertiäre Amine sind solche tertiären Mono- und/oder Polyamine, welche im Molekül pro N-Atom 3 bis 20, vorzugsweise 3 bis 10 C-Atome, enthalten. Besonders bevorzugte tertiäre Amine sind Trimethylamin und Triäthylamin, insbesondere Triäthylamin.

Die Reaktionskomponenten primäres oder sekundäres Carbonsäureamid, offenkettiger α-Halogenalkyläther und tertiäres Amin sind in einem solchen Verhältnis zur Reaktion zu bringen, dass auf 1 mol primäres oder sekundäres Carbonsäureamid etwa 1 mol offenkettiger α-Halogenalkyläther und etwa 1 Äquivalent (= Molekulargewicht: Zahl der N-Atome im Molekül) tertiäres Amin kommen; vorzugsweise wird der α-Halogenalkyläther in einem geringfügigen Überschuss, der zwischen etwa 0,1 und 2 mol/mol Carbonsäureamid betragen kann, und das tertiäre Amin in einer der Menge des α-Halogenalkyläthers äquivalenten Menge eingesetzt. Man kann jedoch auch mit einem Überschuss an Carbonsäureamid arbeiten und nichtumgesetztes Carbonsäureamid dann wieder in die Reaktion zurückführen, was jedoch keine Vorteile bringt.

Wenn für das Verfahren die bevorzugten Ausgangsprodukte der Formeln I und II eingesetzt werden, lässt sich die Umsetzung durch folgende Formelgleichung wiedergeben (wobei für das tertiäre Amin lediglich N< geschrieben ist):

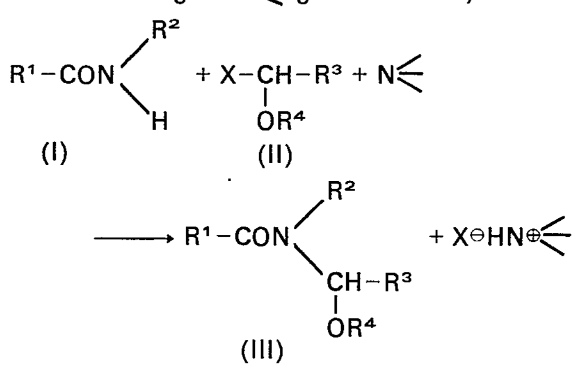

(I) (II) (III)

Die erhaltenen (sekundären und tertiären) N-α-Alkoxyalkylcarbonsäureamide sind dann von der Formel III. In allen Formeln besitzen die Reste $R^1$-$R^4$ und X die vorher bei den Formeln I und II angegebene Bedeutung.

Wenn man als Ausgangscarbonsäureamid ein

primäres Carbonsäureamid verwendet und pro mol desselben nicht 1 mol offenkettigen α-Halogenäthers und 1 Äquivalent tertiäres Amin, sondern etwa 2 mol des α-Halogenäthers und etwa 2 Äquivalente tertiäres Amin einsetzt, kann man auch zu den am N bis-α-alkoxyalkylierten Carbonsäureamiden gelangen. Mit den bevorzugten Ausgangsprodukten der Formel I (worin allerdings $R^2$ hier nur = H sein kann) und II läuft die Umsetzung dann nach folgender Formalgleichung ab:

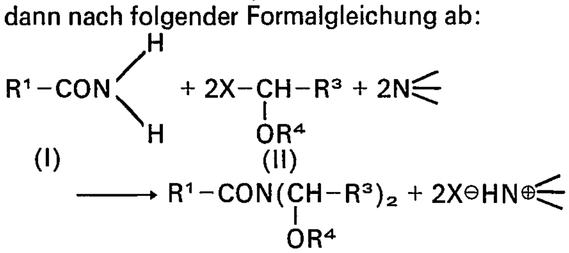

(I) (II)

(IV)

Die erhaltenen N,N-Bis-α-alkoxyalkylcarbonsäureamide besitzen die Formel IV. In allen Formeln besitzen die Reste $R^1$, $R^3$, $R^4$ und X wiederum die vorher bei den Formeln I und II angegebene Bedeutung.

Bei der Ausführung der erfindungsgemässen Umsetzung ist es vorteilhaft, unter Zusatz eines Lösungs- oder Verdünnungsmittels zu arbeiten, da während der Reaktion in der Regel das Halogenid des verwendeten tertiären Amins in fester Form ausfällt. Als Lösungs- oder Verdünnungsmittel können die Reaktionskomponenten selbst — also sowohl das Ausgangscarbonsäureamid als auch der entsprechende offenkettige α-Halogenalkyläther als auch das tertiäre Amin — dienen. Auch das Reaktionsprodukt — also das jeweilige N-α-Alkoxyalkylcarbonsäureamid — kann als Lösungs- oder Verdünnungsmittel verwendet werden.

An anderen Lösungs- oder Verdünnungsmitteln eignen sich praktisch alle inerten organischen Lösungsmittel wie: aliphatische und aromatische Kohlenwasserstoffe (Pentan, Hexan, Octan, Leichtbenzin, Ligroin, Cyclohexan; Benzol, Toluol etc.),
aliphatische und aromatische Halogen-, insbesondere Chlorkohlenwasserstoffe (Methylenchlorid, Tetrachlorkohlenstoff; Chlorbenzol etc.),
aliphatische Äther (Diäthyläther, Diisopropyläther, Tetrahydrofuran etc.),
Ester (Äthylacetat, Propionsäuremethylester etc.),
Ketone (Aceton, Methyläthylketon etc.),
Nitrile (Acetonitril, n-Butyronitril etc.) etc.

Die Wahl des speziellen Lösungs- oder Verdünnungsmittels wird im wesentlichen bestimmt durch die Art der Reaktionsführung und die Aufarbeitung; es ist nicht erforderlich, dass alle Reaktionskomponenten in dem gewählten Lösungs- oder Verdünnungsmittel löslich sind.

Die Reihenfolge der Zugabe der Reaktionskomponenten ist im Prinzip beliebig. Es ist lediglich darauf zu achten, dass nicht das Ausgangscarbonsäureamid und α-Halogenalkyläther in Gegenwart von Säurespuren zusammenkommen, da dann unerwünschte Nebenreaktionen auftreten können. Daher ist es vorteilhaft, dem α-Halogenalkyläther zumindest eine solche Menge tertiäres Amin zuzusetzen, die ausreicht, in dem Äther etwa (von seiner Herstellung her) noch vorhandenen Chlorwasserstoff zu binden, bevor der Äther mit dem Carbonsäureamid vereinigt wird.

Man kann somit also z.B. das Ausgangscarbonsäureamid und das tertiäre Amin vorlegen und den α-Halogenalkyläther hinzudosieren, oder man kann den α-Halogenalkyläther und das tertiäre Amin vorlegen und das Carbonsäureamid hinzudosieren, oder man kann auch den α-Halogenalkyläther vorlegen und das Carbonsäureamid und das tertiäre Amin in beliebiger Reihenfolge zugeben.

Weiterhin ist es möglich, sämtliche Komponenten gleichzeitig in das Reaktionsgefäss einzudosieren.

Besonders vorteilhaft lässt sich die Umsetzung durchführen, wenn man den in bekannter Weise vorzugsweise aus Aldehyd, Alkohol und Halogenwasserstoff hergestellten offenkettigen α-Halogenalkyläther nach Neutralstellung mittels tertiärem Amin in dem gleichen Reaktionsgefäss, in welchem der Äther hergestellt wurde, gleichzeitig getrennt oder in Mischung mit den für die erfindungsgemässe Umsetzung erforderlichen Mengen Carbonsäureamid und tertiärem Amin versetzt, da diese Verfahrensweise die Durchführung der Umsetzung praktisch im Eintopfverfahren erlaubt.

Die Reaktionstemperatur hängt im wesentlichen von der Reaktionsfähigkeit der Komponenten ab. Im allgemeinen kann in einem Temperaturbereich zwischen etwa −20 und etwa +60°C gearbeitet werden. Oberhalb dieses Bereichs fällt die Ausbeute zumeist allmählich ab, während unterhalb des Bereichs die Reaktion meist zu langsam wird und zu lange dauert.

Zur Aufarbeitung des Reaktionsansatzes kann direkt vom entstandenen Hydrohalogenid des eingesetzten tertiären Amins abgesaugt und das Filtrat eingeengt und destilliert werden. Man kann aber dem Ansatz auch Basen — vorzugsweise Alkali- und Erdalkalihydroxide und -carbonate (z.B. NaOH, KOH, $Na_2CO_3$, $Ca(OH)_2$ etc.) — zusetzen und so das tertiäre Amin im Reaktionsgemisch wieder freisetzen. Die Rückgewinnung des Amins ist dann etwa durch Destillation möglich.

Das erfindungsgemässe Verfahren ermöglicht die Herstellung von N-α-Alkoxyalkylcarbonsäureamiden (einschliesslich Lactamen und N,N-Bis-α-alkoxyalkylcarbonsäureamiden) aus einfachen, leicht zugänglichen und daher billigen Ausgangsmaterialien ohne nennenswerten technischen Aufwand und praktisch ohne Umweltbelastung in hohen Ausbeuten und stellt daher einen erheblichen Fortschritt dar.

Darüber hinaus sind die Verfahrensprodukte der nachstehenden Formeln V und VI neue Verbindungen:

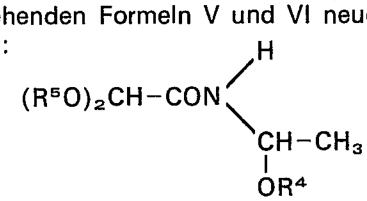

(V)

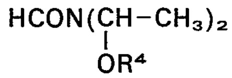 (VI)

In beiden Formeln besitzt $R^4$ die bei Formel II angegebene Bedeutung — d.i. $C_1$ bis $C_4$-Alkyl — wobei hier die Bedeutung

$R^4 = CH_3$, bevorzugt ist, und

$R^5$ (in Formel V) bedeutet $CH_3$ oder $C_2H_5$.

Beispielhafte Verbindungen der Formel V sind:

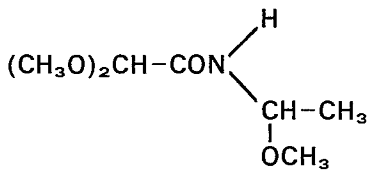

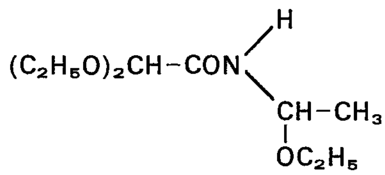

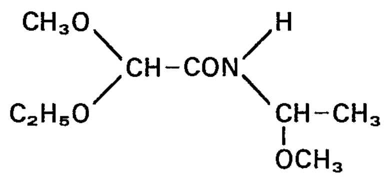

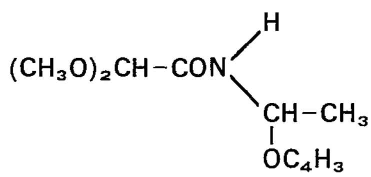 etc.

der Formel VI:

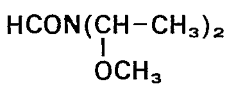 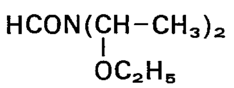 etc.

Die nach dem erfindungsgemässen Verfahren erhaltenen Verbindungen sind Zwischenprodukte hauptsächlich für die Herstellung von N-Alkenyl-, insbesondere N-Vinylcarbonsäureamiden wie z.B. des N-Vinylmethylacetamids, die ihrerseits — wie bereits anfangs erwähnt — in erster Linie zu wertvollen Homo- und Mischpolymerisaten verarbeitet werden können. Die Überführung der N-α-Alkoxyalkylcarbonsäureamide in die entsprechenden N-Alkenyl- bzw. N-Vinylcarbonsäureamide geschieht auf bekannte Weise vorzugsweise durch Erhitzen auf Temperaturen von etwa 60 bis 350°C, meist in Gegenwart von Katalysatoren (DE-PS Nr. 1235893, US-PS Nr. 3377340, GB-PS Nr. 1125324, DE-OS Nr. 2336977 etc.). Entsprechendes gilt auch für die N,N-Bis-α-alkoxyalkylcarbonsäureamide.

Die durch Alkanolabspaltung aus den N-α-Alkoxyäthylverbindungen der Formel V resultierenden N-Vinylverbindungen sind ebenfalls neu; sie besitzen die Formel VII:

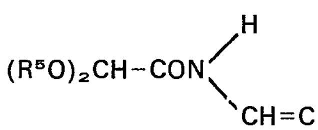 (VII)

worin $R^5$ die gleiche Bedeutung wie in Formel V besitzt.

Unter Formel VII fallen also:

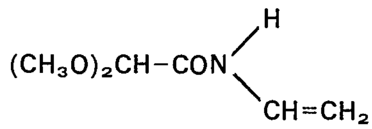

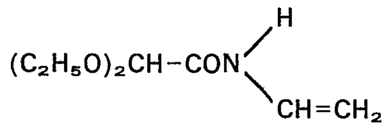

und

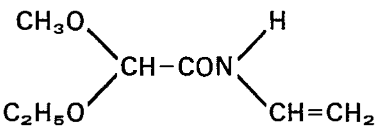

Die Erfindung wird nun durch die folgenden Beispiele näher erläutert. Den Erfindungsbeispielen A folgen 2 Beispiele für die Alkoholabspaltung aus nach dem erfindungsgemässen Verfahren erhaltenen Produkten zu den entsprechenden N-Vinylprodukten B sowie ein Vergleichsbeispiel C, welches zeigt, dass die aus „Tetrahedron Letters" 1976, 1725 bis 1728, bekannte Reaktion von Pyridon-2 mit α-Chlortetrahydrofuran nicht mehr geht, wenn man das Pyridon-2 durch ein normales Carbonsäureamid ersetzt.

A) *Erfindungsbeispiele*

*Beispiel 1*

*N-α-Methoxyäthyl-N-äthylformamid*

Zu einer Mischung von 60 ml Leichtbenzin und 38 g (0,4 mol) α-Chloräthylmethyläther tropft man bei 0°C 8,0 g Triäthylamin und danach eine Mischung von 42,5 g Triäthylamin (insgesamt 0,5 mol) und 14,6 g (0,2 mol) N-Äthylformamid.

Nach 2 h saugt man eiskalt ab, wäscht den Filterkuchen mit Hexan und engt das Filtrat im Vakuum ein. Der Rückstand wird flash-destilliert. Man erhält 19,8 g (76% d.Th.) N-α-Methoxyäthyl-N-äthylformamid. $Kp._{20}$: 89-91°C.

1H-NMR ($CDCl_3$): 1,15 ppm (t); 1,4 ppm (d); 3,15 ppm (s); 3,25 ppm (q); 4,6 ppm (q); 5,55 ppm (q); 8,12 ppm (s); 8,22 ppm (s).

*Beispiel 2*

*N-α-Methoxyäthylacetamid*

Zu einer Mischung von 30 ml Acetonitril und 38 g (0,4 mol) α-Chloräthylmethyläther tropft man bei −10°C zunächst 7,3 g Triäthylamin und dann gleichzeitig aus zwei Tropftrichtern 11,8 g (0,2 mol) Acetamid, gelöst in 30 ml Acetonitril und 44 g Triäthylamin (Gesamtmenge: 0,5 mol). 2 h nach beendetem Eintropfen saugt man ab, entfernt das Lösungsmittel im Vakuum, extrahiert den Rückstand mit i-Propyläther und rotiert erneut ein. Der Rückstand wird flash-destilliert. Ausbeute 17,6 g (75% d.Th.) N-α-Methoxyäthylacetamid. $Kp._{0,2}$: 60-62°C.

$^1$H'-NMR ($CDCl_3$): 1,3 ppm (d); 2,0 ppm (s); 3,3 ppm (s); 5,0-5,5 ppm (m); 6,25 ppm (breit).

*Beispiel 3*

*N-α-(i-Butoxy)äthyl-N-methylacetamid*

Zu einer Mischung von 136,5 g (1 mol) i-Butyl-α-chloräthyläther, 130 ml Hexan und 20 g Triäthylamin tropft man bei 0°C eine Mischung von 91 g Triäthylamin (insgesamt 1,1 mol) und 36,5 g (0,5 mol) N-Methylacetamid.

2 h nach beendetem Eintropfen setzt man bei 0°C 40 g schuppenförmiges, technisches Natriumhydroxid und 20 ml 50%ige Natronlauge in kleinen Portionen zu, rührt 30 min nach, dekantiert und spült den Kolben mit Hexan nach. Von der filtrierten Hexanlösung wird das Lösungsmittel und das freigesetzte Triäthylamin wird das Lösungsmittel und das freigesetzte Triäthylamin im Vakuum abgezogen und der Rückstand im Vakuum destilliert. Ausbeute: 58 g (67% d.Th.). $Kp._2$: 56°C.

1H-NMR ($CDCl_3$): 1,0 ppm (d); 2,25 ppm (dd); 1,6-2,0 ppm (m); 2,15 ppm (d); 2,85 ppm (d); 3,15 ppm (d); 5,1 ppm (q); 5,9 ppm (q).

*Beispiel 4*

*N-α-Methoxyäthyl-N-methylacetamid*

Zu einer Lösung von 94,5 g (1 mol) α-Chloräthylmethyläther in 200 ml Hexan tropft man bei 0°C zunächst 20 g Triäthylamin und danach eine Mischung von 91 g Triäthylamin (insgesamt 1,1 mol) und 36,5 g (0,5 mol) N-Methylacetamid. Nach 2 h setzt man unter Eiskühlung 44 g (1,1 mol) schuppenförmiges technisches Natriumhydroxid und tropfenweise 20 ml 50%ige Natronlauge zu und rührt noch ½ h. Man dekantiert die Hexanlösung, spült den Kolben im Destillat das eingesetzte Triäthylamin vollständig zurückgewonnen wird. Der Rückstand wird flash-destilliert im Vakuum. Man erhält 61 g (93% d.Th.) N-α-Methoxyäthyl-N-methylacetamid. $Kp._{23}$: 84°C.

1H-NMR ($CDCl_3$): 1,2-1,4 ppm (dd); 2,1 ppm (d); 2,8 ppm (d); 3,2 ppm (d); 4,95-5,15 ppm (q); 5,6-5,9 ppm (q).

*Beispiel 5*

*N-α-Äthoxyäthyl-N-methylacetamid*

Zu einer Mischung von 44 g α-Chlordiäthyläther (0,4 mol) und 100 ml Hexan tropft man bei 60°C innerhalb von 15 min eine Mischung von 50 g (0,5 mol) Triäthylamin und 14,6 g (0,2 mol) N-Methylacetamid.

Nach weiteren 15 min kühlt man in Eis, saugt ab, wäscht den Filterkuchen mit Hexan und destilliert das Reaktionsprodukt nach Abziehen des Lösungsmittels im Vakuum. Man erhält 23,2 g (80% d.Th.) N-α-Äthoxyäthyl-N-methylacetamid. $Kp._{23}$: 94-95°C.

1H-NMR ($CDCl_3$): 1,0-1,4 ppm (m); 2,1 ppm (d); 2,8 ppm (d); 3,3-3,6 ppm (q); 4,9-5,2 ppm (q); 5,7-6,0 ppm (q).

*Beispiel 6*

*N-α-Methoxyäthyl-N-methylacetamid, aus Acetaldehyd, Methanol und N-Methylacetamid*

In eine Mischung von 49 g (1,1 Äquivalente) Paraldehyd und 35 g (1,1 mol) Methanol leitet man bei 0°C 59 g (1,6 mol) trockenen Chlorwasserstoff ein.

Man setzt 160 ml Leichtbenzin zu und entfernt die untere, wässerige Schicht aus dem Reaktionsgefäss. Die obere Schicht wird durch Einleiten von Stickstoff von überschüssigem Chlorwasserstoff befreit. In diese tropft man bei 0°C zunächst 20 g Triäthylamin und danach eine Mischung von 91 g Triäthylamin (insgesamt 1,1 mol) und 36,5 g (0,5 mol) Methylacetamid zu. Nach 2 h setzt man unter Eiskühlung 44 g (1,1 mol) schuppenförmiges Natriumhydroxid und dann tropfenweise 20 ml 50%ige Natronlauge zu und rührt weitere 30 min. Man dekantiert die Hexanlösung, spült das Reaktionsgefäss mit Hexan nach und engt die Reaktionslösung ein, wobei Triäthylamin verlustlos zurückgewonnen wird. Der Rückstand wird flash-destilliert. Ausbeute: 59 g (90% d.Th.). Zur Überführung in N-Vinyl-N-methylacetamid kann das undestillierte Rohprodukt direkt in den Spaltofen von Beispiel B hineindestilliert werden.

*Beispiel 7*

*N-α-Methoxy-i-butyl-N-methylacetamid*

Zu einer Mischung von 49 g (0,4 mol) α-Chlor-i-butylmethyläther und 50 ml Leichtbenzin tropft man unter Eiskühlung zunächst 15 g Triäthylamin und danach eine Mischung von 35,5 g Triäthylamin und 14,6 g (0,2 mol) N-Methylacetamid.

Nach beendeter Reaktion setzt man 20 g festes Natriumhydroxid und 10 ml 50%ige Natronlauge zu, man rührt nach 30 min, saugt ab und destilliert das Reaktionsprodukt nach Abtreiben von Triäthylamin und Lösungsmittel. Man erhält 23,3 g (73% d.Th.) N-α-Methoxy-i-butyl-N-methylacetamid. $Kp._{0,05}$: 53°C.

*Beispiel 8*

*1-α-Methoxyäthyl-4,4-dimethylazetidin-2-on*

Zu einer Lösung von 38 g (0,4 mol) α-Chloräthylmethyläther in 60 ml Hexan tropft man bei 0°C 7,3 g Triäthylamin und danach eine Mischung von 19,8 g (0,2 mol) 4,4-Dimethylazetidin-2-on und 43,2 g Triäthylamin. Man rührt 2 h bei 0°C nach und setzt 10 ml 50%ige Natronlauge und 20 g gepulvertes Natriumhydroxid zu. Nach weiteren 30 min dekantiert man, wäscht den Bodensatz mit Hexan nach und rotiert die Hexanauszüge ein. Man erhält 25,5 g (81% d.Th.) 1-α-Methoxyäthyl-4,4-dimethylazetidin-2-on.

1H-NMR ($CDCl_3$): 1,4 ppm (d); 1,5 ppm (s); 2,75 ppm (s); 3,3 ppm (s); 4,8-5,1 ppm (q).

*Beispiel 9*

*N-α-Methoxyäthylpyrrolidon*

Zu einer Lösung von 38 g (0,4 mol) α-Chloräthylmethyläther in 30 ml Methylenchlorid tropft man bei 0°C zunächst 7,3 g Triäthylamin und danach gleichzeitig aus zwei Tropftrichtern 43,2 g Triäthylamin und eine Lösung von 17 g (0,2 mol) Pyrrolidon in 40 ml Methylenchlorid.

2 h nach beendetem Eintropfen verrührt man 1 h mit einer Aufschlämmung von 30 g gepulvertem

Kaliumhydroxid in 15 ml 50%iger Kalilauge. Man dekantiert, extrahiert den Rückstand mit Methylenchlorid und engt die Methylenchloridlösung im Vakuum ein. Man erhält 22,3 g (78% d.Th.) N-α-Methoxyäthylpyrrolidon.

1H-NMR ($CDCl_3$): 1,3 ppm (d); 1,8-2,6 ppm (m); 3,2 ppm (s); 3,1-3,5 ppm (m); 5,1-5,5 ppm (q).

*Beispiel 10*

*α,α-Dimethoxyessigsäure-N-α'-methoxyäthylamid*

In die Mischung von 176 g (4 val) Paraldehyd und 144 g (4,4 mol) Methanol leitet man bei 10°C 200 g gasförmigen Chlorwasserstoff ein. Nach Abtrennen der unteren Flüssigkeitsschicht wird die obere durch Einleiten von trockenem Stickstoff von überschüssigem Chlorwasserstoff befreit. Man gibt 200 ml Acetonitril und 47 g Triäthylamin zu. Anschliessend tropft man bei 0 bis 10°C gleichzeitig 454,2 g (4,4 mol) Triäthylamin und eine Lösung von 238 g (2 mol) Dimethoxyessigsäureamid in 380 ml Acetonitril zu. Nach beendetem Zutropfen rührt man 3 h bei Raumtemperatur und 2 h bei 50°C nach. Bei 20°C versetzt man dann den Ansatz mit 720 mg (6 mol) 33%iger Natronlauge und rührt 30 min. Dann wird die untere Schicht abgetrennt und 2mal mit Acetonitril extrahiert. Die vereinigten Acetonitrillösungen werden einrotiert, der Rückstand im Vakuum destilliert. Man erhält 325 g (92% d.Th.) α,α-Dimethoxyessigsäure-N-α'-methoxyäthylamid. $Kp._{0,7}$: 78-82°C.

$^1$H-NMR ($CDCl_3$): 1,35 ppm (d, j = 6 Hz); 3,3 ppm (s); 3,4 ppm (s); 4,7 ppm (s); 5,1-5,5 ppm (m); 6,8 ppm (breit).

Intensitätsverhältnis 3:3:6:1:1:1.

*Analyse* für $C_7H_{15}NO_4$ (177,20):

| | | | |
|---|---|---|---|
| berechnet: | C 47,44 | H 8,5 | N 7,90% |
| gefunden: | C 47,7 | H 8,5 | N 7,7 % |

Das als Ausgangsmaterial eingesetzte α,α-Dimethoxyessigsäureamid wurde aus α,α-Dimethoxyessigsäuremethylester durch Umsetzung mit wässerigem Ammoniak erhalten. Fp.: 69-70°C (Äthylacetat).

*Beispiel 11*

*N,N-Di-(α-Methoxyäthyl)formamid und N-α-Methoxyäthylformamid*

Man erzeugt wie in Beispiel 10 aus 2 val Paraldehyd, 2,2 mol Methanol und 2,7 mol Chlorwasserstoff den α-Chloräthylmethyläther. Dieser wird mit 100 ml Acetonitril verdünnt. Anschliessend tropft man bei 0°C gleichzeitig 250,3 g (2,5 mol) Triäthylamin und eine Lösung von 45 g (1 mol) Formamid in 265 ml Acetonitril zu.

Nach beendetem Zutropfen rührt man 3 h bei Raumtemperatur und 2 h bei 50°C nach, verrührt mit 360 g (3 mol) 33%iger Natronlauge 30 min, trennt ab, extrahiert die wässerige Schicht 2mal mit Acetonitril und rotiert die Acetonitrillösung ein.

Das Rohprodukt der Reaktion extrahiert man mit Hexan. Der hexanlösliche Anteil wird nach Abdestillieren des Lösungsmittels im Vakuum destilliert. Man erhält 83 g (50% d.Th.) N,N-Di-(α-methoxyäthyl)formamid. $Fp._{0,1}$: 50-52°C.

$^1$H-NMR ($CDCl_3$): 1,2-1,6 ppm (m); 3,2-3,4 ppm (m); 4,5-4,8 ppm (m); 5,5-5,8 ppm (m); 8,45-8,55 ppm (s).

Intensitätsverhältnis: 6:6:1:1:1.

Durch Fraktionieren des hexanunlöslichen Rückstandes des rohen Reaktionsproduktes erhält man 24 g (23% d.Th.) N-α-Methoxyäthylformamid. $Kp._{0,5}$: 60-62°C.

Das Produkt ist nach 1H-NMR-Spektrum und Dünnschichtchromatographie (Silicagel, Laufmittel: 90% Chloroform, 10% Methanol) identisch mit N-α-Methoxyäthylformamid, das elektrochemisch gemäss EP-A Nr. 0019225, Beispiel 1 bis 7, Tabelle 1, hergestellt wurde.

Legt man bei der Reaktion ein Gemisch von 90 g (2 ml) Formamid, 253 g (2,5 mol) Diäthylamin und 341 ml Acetonitril vor und tropft bei Raumtemperatur 189 g (2 mol) α-Chlorethylmethyläther zu, so erhält man bei gleicher Aufarbeitung des Ansatzes N-α-Methoxyäthylformamid als Hauptprodukt.

B) *Alkoholabspaltung*

*Beispiel 1*

*N-Vinyl-N-methylacetamid*

In einem Dünnschichtverdampfer wird unter Stickstoffüberlagerung bei 270°C N-α-Methoxyäthyl-N-methylacetamid verdampft. Der Dampf wird durch den mit einer porösen Kieselsäure als Katalysator gefüllten Ofen geleitet. Die Spaltprodukte werden am Ende Ofens kondensiert. Tabelle 1 zeigt den Gehalt an nichtgespaltenem Ausgangsmaterial im Kondensat in Abhängigkeit von der Ofentemperatur bei einem Durchsatz von 400 g Ausgangsmaterial/h.

| Temp. (°C) | N-α-Methoxyäthyl-N-methylacetamid, Restgehalt (%) |
|---|---|
| 225 | 3,3 |
| 240 | 2,0 |
| 250 | 1,7 |
| 265 | 0,4 |
| 280 | 0,3 |
| 290 | 0,3 |

Die Gesamtausbeuten an N-Vinyl-N-methylacetamid und nicht gespaltenem Ausgangsmaterial liegen bei 97-98%.

Das rohe Spaltmaterial wird in einer Füllkörperkolonne rektifiziert. Nach Abdestillieren des abgespaltenen Methanols erhält man in quantitativer Ausbeute N-Vinyl-N-methylacetamid. $Kp._{11}$: 51,5°C.

*Beispiel 2*

*α,α-Dimethoxyessigsäure-N-Vinylamid*

In gleicher Weise wie in vorstehendem Beispiel wird α,α-Dimethoxyessigsäure-N-α-methoxyäthylamid (Herstellung s. Beispiel 10) bei 300°C in α,α-Dimethoxyessigsäure-N-vinylamid überführt. Bei einem Umsatz von über 99% beträgt die Ausbeute 95% d.Th.

Man erhält die Vinylverbindung in gleicher Ausbeute, wenn man bei einer Ofentemperatur von ebenfalls 300°C die Reaktion bei 100 Torr durchführt. Der Verdampfer wird in diesem Fall nur auf 220°C geheizt.

α,α-Dimethoxyessigsäure-N-vinylamid siedet bei 64 °C/0,1 Torr.

NMR-Spektrum ($CDCl_3$): 3,4 ppm (s); 4,4-4,8 ppm (2 d); 4,7 ppm (s); 6,6-7,3 ppm (m); 8,2 ppm (breit).

Intensitätsverhältnis: 6:2:1:1:1.

C) *Vergleichsbeispiel*

In 40 ml wasserfreiem Tetrahydrofuran löst man 5,4 g (40 mmol) Sulfurylchlorid. Nach beendeter Reaktion gibt man zunächst die Hälfte dieser Lösung zu einer Mischung von 1,46 g (20 mmol) N-Methylacetamid und 9,09 g (90 mmol) wasserfreiem Triäthylamin in 40 ml wasserfreiem Tetrahydrofuran.

Nach 15 min setzt man den Rest der abreagierten Sulfurylchloridlösung zu. Nach weiteren 15 min kühlt man in Eis, saugt ab, rotiert das Filtrat ein, extrahiert den Rückstand mit Leichtbenzin, trocknet den Extrakt über $MgSO_4$, rotiert erneut ein und destilliert den Rückstand (1,12 g) im Vakuum. Man erhält neben verharztem nicht destilliertem Rückstand 0,22 g uneinheitliches Destillat, das nach $^1H$-NMR (breites Signal bei 7,3 ppm) und IR-Spektrum (Amid-II-Bande bei 1540 $cm^{-1}$, NH-Bande bei 3330 $cm^{-1}$) im wesentlichen aus Amiden besteht, die am Stickstoffatom noch ein Wasserstoffatom tragen.

## Patentansprüche

1. Verfahren zur Herstellung von N-α-Alkoxyalkylcarbonsäureamiden, ausgehend von primären oder sekundären Carbonsäureamiden, dadurch gekennzeichnet, dass man primäre oder sekundäre Amide aliphatischer, araliphatischer oder aromatischer Carbonsäuren oder cyclische Carbonsäureamide (Lactame), welche nicht zur Ausbildung eines aromatischen Systems befähigt sind, mit offenkettigen α-Halogenalkyläthern einer C-Atomzahl von mindestens 3 pro Molekühl in Gegenwart von tertiären Aminen umsetzt.

2. Verfahren nach Anspruch 1, dadurch gekennzeichnet, dass man als primäre oder sekundäre Carbonsäureamide Verbindungen der Formel (I)

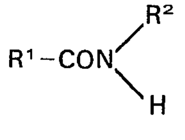 (I)

worin

$R^1$ = H oder ggf. durch reaktionsinerte Gruppen substituiertes $C_1$ bis $C_4$-Alkyl,

$R^2$ = H oder $C_1$ bis $C_3$-Alkyl, oder

$R^1 + R^2$ = zusammen eine — ggf. durch reaktionsinerte Gruppen substituierte — Alkylengruppe mit 2 bis 6 C-Atomen in der Kette, verwendet.

3. Verfahren nach Ansprüchen 1 und 2, dadurch gekennzeichnet, dass man als primäre oder sekundäre Carbonsäureamide Verbindungen der Formel (I) mit $R^1$ = H, $CH_3$, $C_2H_5$ und $R^2$ = H, $CH_3$, $C_2H_5$ verwendet.

4. Verfahren nach Ansprüchen 1 bis 3, dadurch gekennzeichnet, dass man als offenkettige α-Halogenalkyläther mit einer C-Atomzahl von mindestens 3 pro Molekül Verbindungen der Formel (II)

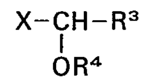 (II)

worin

$R^3$ = $C_1$ bis $C_4$-Alkyl,

$R^4$ = ebenfalls $C_1$ bis $C_4$-Alkyl, und

X = Cl oder Br, vorzugsweise Cl bedeuten, verwendet.

5. Verfahren nach Ansprüchen 1 bis 4, dadurch gekennzeichnet, dass man als tertiäre Amine solche tertiären Mono- oder Polyamine verwendet, welche im Molekül pro N-Atom 3 bis 20, vorzugsweise 3 bis 10 C-Atome enthalten.

6. Verfahren nach Ansprüchen 1 bis 5, dadurch gekennzeichnet, dass man als tertiäre Amine Trimethyl- und/oder Triäthylamin, vorzugsweise Triäthylamin, verwendet.

7. Verfahren nach Ansprüchen 1 bis 6, dadurch gekennzeichnet, dass man die offenkettigen α-Halogenalkyläther in bekannter Weise *in situ* herstellt aus Aldehyd, Alkohol und Halogenwasserstoff und nach Neutralstellen der Reaktionslösung mittels tertiärem Amin das primäre oder sekundäre Carbonsäureamid und weiteres tertiäres Amin hinzudosiert.

8. Verfahren nach Ansprüchen 1 bis 7, dadurch gekennzeichnet, dass man die Umsetzung im Temperaturbereich zwischen etwa −20 und +60°C durchführt.

9. Verbindungen der formel (V):

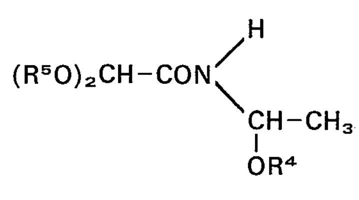 (V)

worin

$R^4$ = $C_1$ bis $C_4$-Alkyl, vorzugsweise $CH_3$ und

$R^5$ = $CH_3$ oder $C_2H_5$.

10. Verbindungen der Formel (VI):

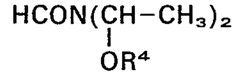 (VI)

worin $R^4$ die gleiche Bedeutung wie in Formel (V) besitzt.

11. Verbindungen der Formel (VII):

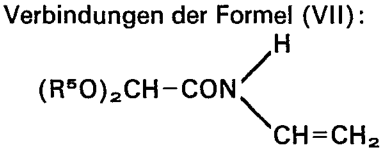
(VII)

worin $R^5$ die gleiche Bedeutung wie in Formel (V) besitzt (= $CH_3$ oder $C_2H_5$).

## Revendications

1. Procédé pour préparer des N-α-alcoxyalkylcarboxamides à partir de carboxamides primaires ou secondaires, procédé caractérisé en ce qu'on fait réagir des amides primaires ou secondaires d'acides carboxyliques aliphatiques, araliphatiques ou aromatiques, ou des carboxamides cycliques (lactames) inaptes à former un système aromatique, avec des éthers α-halogénoalkyliques à chaîne ouverte qui contiennent au moins trois atomes de carbone par molécule, en présence d'amines tertiaires.

2. Procédé selon la revendication 1, caractérisé en ce qu'on utilise, comme carboxamides primaires ou secondaires, des composés répondant à la formule (I):

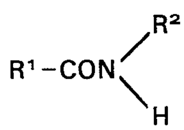
(I)

dans laquelle:

$R^1$ représente l'hydrogène ou un radical alkyle en $C_1$-$C_4$ éventuellement porteur de substituants inertes à l'égard de la réaction,

$R^2$ représente l'hydrogène ou un radical alkyle contenant de 1 à 3 atomes de carbone, ou

$R^1$ et $R^2$ forment ensemble un radical alkylène qui contient de 2 à 6 atomes de carbone dans sa chaîne et qui peut éventuellement porter des substituants inertes à l'égard de la réaction.

3. Procédé selon les revendications 1 et 2, caractérisé en ce qu'on utilise, comme carboxamides primaires ou secondaires, des composés de formule (I) dans lesquels $R^1$ représente l'hydrogène ou un radical $CH_3$ ou $C_2H_5$ et $R^2$ représente l'hydrogène ou un radical $CH_3$ ou $C_2H_5$.

4. Procédé selon l'une quelconque des revendications 1 à 3, caractérisé en ce qu'on utilise, comme éthers α-halogénoalkyliques à chaîne ouverte qui contiennent au moins trois atomes de carbone par molécule, des composés répondant à la formule (II):

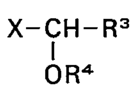
(II)

dans laquelle

$R^3$ représente un radical alkyle contenant de 1 à 4 atomes de carbone,

$R^4$ représente également un radical alkyle contenant de 1 à 4 atomes de carbone, et

X représente Cl ou Br, de préférence Cl.

5. Procédé selon l'une quelconque des revendications 1 à 4, caractérisé en ce qu'on utilise, comme amines tertiaires, des monoamines ou des polyamines tertiaires qui contiennent dans leur molécule, par atome d'azote, de 3 à 20 atomes de carbone, de préférence de 3 à 10.

6. Procédé selon l'une quelconque des revendications 1 à 5, caractérisé en ce qu'on utilise, comme amines tertiaires, la triméthylamine et/ou la triéthylamine, de préférence la triéthylamine.

7. Procédé selon l'une quelconque des revendications 1 à 6, caractérisé en ce que les éthers α-halogénoalkyliques à chaîne ouverte sont préparés de manière connue, *in situ*, à partir de l'aldéhyde, de l'alcool et de l'halogénure d'hydrogène et, après neutralisation de la solution réactionnelle au moyen d'une amine tertiaire, on y ajoute progressivement le carboxamide primaire ou secondaire et une quantité supplémentaire de l'amine tertiaire.

8. Procédé selon l'une quelconque des revendications 1 à 7, caractérisé en ce que la réaction est effectuée dans un intervalle de température allant d'environ −20 à +60°C.

9. Composés répondant à la formule (V):

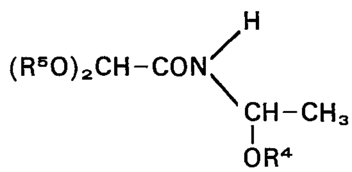
(V)

dans laquelle

$R^4$ représente un radical alkyle contenant de 1 à 4 atomes de carbone, de préférence $CH_3$, et

$R^5$ représente $CH_3$ ou $C_2H_5$.

10. Composés répondant à la formule (VI):

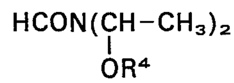
(VI)

dans laquelle $R^4$ a la même signification que dans la formule (V).

11. Composés répondant à la formule (VII):

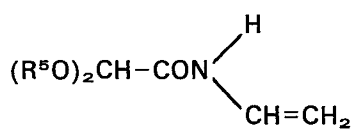
(VII)

dans laquelle $R^5$ a la même signification que dans la formule (V) (c'est-à-dire $CH_3$ ou $C_2H_5$).

## Claims

1. A process for the preparation of N-α-alkoxyalkylcarboxamides starting from primary or secondary carboxamides, characterized in reacting primary or secondary amides of aliphatic, araliphatic or aromatic carboxylic acids, or cyclic carboxamides (lactams) which are not capable of forming an aromatic system, with open-chain α-halogenoalkyl ethers with at least 3 C atoms per molecule, in the presence of tertiary amines.

2. A process as claimed in claim 1, characterized in using as the primary or secondary carboxamides compounds of the formula (I):

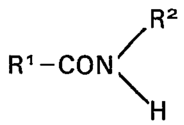 (I)

wherein

$R^1$ = H or $C_1$-$C_4$ alkyl optionally substituted by groups which are inert towards the reaction,

$R^2$ = H or $C_1$-$C_3$ alkyl, or

$R^1$ + $R^2$ together = an alkylene group with 2-6 C atoms in the chain, optionally substituted by groups which are inert towards the reaction.

3. A process as claimed in claims 1 and 2, characterized in using as the primary or secondary carboxamides compounds of the formula (I) in which $R^1$ = H, $CH_3$ or $C_2H_5$ and $R^2$ = H, $CH_3$ or $C_2H_5$.

4. A process as claimed in claims 1 to 3, characterized in using as the open-chain α-halogenoalkyl ethers with at least 3 C atoms per molecule compounds of the formula (II):

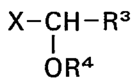 (II)

wherein

$R^3$ = $C_1$-$C_4$ alkyl,

$R_4$ also = $C_1$-$C_4$ alkyl, and

X = Cl or Br, preferably Cl.

5. A process as claimed in claims 1 to 4, characterized in using as the tertiary amines those tertiary monoamines or polyamines which contain 3 to 20, preferably 3 to 10, C atoms in the molecule per N atom.

6. A process as claimed in claims 1 to 5, characterized in using as the tertiary amines trimethylamine and/or triethylamine, preferably triethylamine.

7. A process as claimed in claims 1 to 6, characterized in that the open-chain α-halogenoalkyl ethers are prepared *in situ*, in a known manner, from aldehyde, alcohol and hydrogen halide and, after neutralization of the reaction solution by means of tertiary amine, the primary or secondary carboxamide and additional tertiary amine are metered in.

8. A process as claimed in claims 1 to 7, characterized in carrying out the reaction in the temperature range between about −20 and +60°C.

9. Compounds of the formula (V):

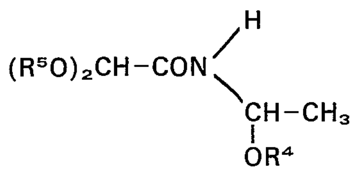 (V)

wherein

$R^4$ = $C_1$-$C_4$ alkyl, preferably $CH_3$, and

$R^5$ = $CH_3$ or $C_2H_5$.

10. Compounds of the formula (VI):

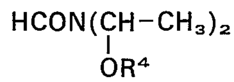 (VI)

wherein $R^4$ has the same meaning as in formula (V).

11. Compounds of the formula (VII):

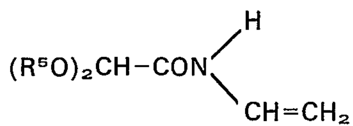 (VII)

wherein $R^5$ has the same meaning as in formula (V) (= $CH_3$ or $C_2H_5$).